(19) Europäisches Patentamt
European Patent Office
Office européen des brevets

(11) Veröffentlichungsnummer: **0 094 564**
**B1**

(12) **EUROPÄISCHE PATENTSCHRIFT**

(45) Veröffentlichungstag der Patentschrift: **30.09.87**

(21) Anmeldenummer: **83104412.8**

(22) Anmeldetag: **05.05.83**

(51) Int. Cl.⁴: **C 07 D 405/06,**
C 07 D 405/14,
A 01 N 43/653, A 01 N 43/50,
A 61 K 31/41 // C07C25/24,
C07D303/08

# (54) Azolylmethyloxirane, ihre Herstellung und Verwendung als Arznei- und Pflanzenschutzmittel.

(30) Priorität: **14.05.82 DE 3218129**
**14.05.82 DE 3218130**

(43) Veröffentlichungstag der Anmeldung:
**23.11.83 Patentblatt 83/47**

(45) Bekanntmachung des Hinweises auf die Patenterteilung:
**30.09.87 Patentblatt 87/40**

(84) Benannte Vertragsstaaten:
**AT BE CH DE FR GB IT LI NL SE**

(56) Entgegenhaltungen:
**EP-A-0 023 103**
**EP-A-0 061 051**

**Tetrabedron Letters 1977, no. 50, pages 4397-4400**

(73) Patentinhaber: **BASF Aktiengesellschaft**
**Carl-Bosch-Strasse 38**
**D-6700 Ludwigshafen (DE)**

(72) Erfinder: **Janssen, Bernd, Dr.**
**Leuschnerstrasse 18a**
**D-6700 Ludwigshafen (DE)**
Erfinder: **Meyer, Norbert, Dr.**
**Stahlbuehlring 155a**
**D-6802 Ladenburg (DE)**
Erfinder: **Pommer, Ernst-Heinrich, Dr.**
**Berliner Platz 7**
**D-6703 Limburgerhof (DE)**
Erfinder: **Ammermann, Eberhard, Dr.**
**Sachsenstrasse 3**
**D-6700 Ludwigshafen (DE)**
Erfinder: **Kohlmann, Friedrich-Wilhelm, Dr.**
**An der Duene 6**
**D-2082 Moorrege (DE)**
Erfinder: **Wesenberg, Walter**
**Dorfstrasse 16**
**D-2421 Bujendorf ueber Eutin (DE)**
Erfinder: **Heberle, Wolfgang, Dr.**
**An der Duene 4**
**D-2082 Moorrege (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 094 564 B1

Courier Press, Leamington Spa, England.

**Beschreibung**

Die Erfindung betrifft neue Azolverbindungen, Verfahren zu deren Herstellung, diese enthaltende therapeutische Mittel, die als Antimykotika verwendet werden können und deren Verwendung bei der Heilung von Krankheiten sowie Fungizide, die diese Verbindungen enthalten.

Es sind zahlreiche antimykotische Mittel, wie z.B. Miconazol (DE—OS 19 40 388) bekannt. Ihre Wirkungen befriedigen nicht immer (Lit.: Chemotherapy *22,* 1 (1976)); Dtsch. Apoth. Ztg. *118,* 1269 (1978); Z. Hautkr. *56,* 1109 (1981)). Es ist weiter bekannt, Azolverbindungen, z.B. Azolylmethylcarbinole oder Azolylmethylketone (DE—OS 24 31 407, FR—PS 2 249 616) als Fungizide zu verwenden. Der Vorliegenden Erfindung liegt die Aufgabe zugrunde, neue und wirksamere Antimykotika bzw. Fungizide zur Verfügung zu stellen.

Es wurde nun gefunden, daß Verbindungen der Formel I

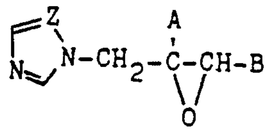

(I)

in der

A und B gleich oder verschieden sind und Naphthyl, Biphenyl oder Phenyl bedeuten, wobei der Phenylrest durch Halogen, Nitro, Alkyl, Alkoxy oder Halogenalkyl mit jeweils 1 bis 4 Kohlenstoffatomen, Phenoxy sowie den Phenylsulfonylrest substituiert sein kann.

Z für die CH-Gruppe oder ein Stickstoffatom steht sowie deren physiologisch bzw. für Pflanzen verträgliche Säureadditions-Salze gute antimikrobielle, insbesondere antimykotische, und fungizide Eigenschaften aufweisen.

Die neuen Verbindungen der Formel I enthalten chirale Zentren und werden im allgemeinen in Form von Racematen oder als Diastereomerengemische von erythro- sowie threo-Formen erhalten. Die erythro- und threo-Diastereomeren lassen sich bei den erfindungsgemäßen Verbindungen beispielsweise aufgrund ihrer unterschiedlichen Löslichkeit oder durch Säulenchromatographie trennen und in reiner Form isolieren. Aus solchen einheitlichen Diastereomerenpaaren kann man mit bekannten Methoden einheitliche Enantiomere erhalten. Als antimikrobielle Mittel kann man sowohl die einheitlichen Diastereomere bzw. Enantiomere wie auch deren bei der Synthese anfallende Gemische verwenden. Entsprechendes gilt für die fungiziden Mittel.

A und B bedeuten beispielsweise: 1-Naphthyl, 2-Naphthyl, p-Biphenyl, Phenyl, 2-Chlorphenyl, 3-Chlorphenyl, 4-Chlorphenyl, 4-Fluorphenyl, 4-Bromphenyl, 2,4-Dichlorphenyl, 3,4-Dichlorphenyl, 3,5-Dichlorphenyl, 3-Chlor-4-methylphenyl, 2-Methoxyphenyl, 3-Methoxyphenyl, 2,4-Dimethoxyphenyl, 3,4-Dimethoxyphenyl, 4-Methoxyphenyl, 4-Ethoxyphenyl, 4-tert.-Butoxyphenyl, 4-Methylphenyl, 4-Ethylphenyl, 4-Isopropylphenyl, 4-tert.-Butylphenyl, 4-Phenoxyphenyl, 3-Phenoxyphenyl, 3-Nitrophenyl, 4-Nitrophenyl, 3-Trifluormethylphenyl, 4-Trifluormethylphenyl und 4-Phenylsulfonylphenyl.

Bevorzugt sind: Phenyl, 4-Chlorphenyl, 4-Bromphenyl, 2,4-Dichlorphenyl und 4-tert.-Butylphenyl.

Als übliche Säuren zur Bildung physiologisch verträglicher Salze kommen vorzugsweise Halogenwasserstoffsäuren, wie z.B. Chlorwasserstoffsäure und Bromwasserstoffsäure, insbesondere die Chlorwasserstoffsäure, mit der die erfindungsgemäßen Verbindungen besonders gut kristallisierende Salze bilden, in Frage, ferner Phosphorsäure, Salpetersäure, Schwefelsäure, mono- und bifunktionelle Carbonsäuren und Hydroxycarbonsäuren, wie Essigsäure, Oxalsäure, Maleinsäure, Bernsteinsäure, Fumarsäure, Weinsäure, Zitronensäure, Salicylsäure, Sorbinsäure, Milchsäure sowie Sulfonsäuren, P-Toluolsulfonsäure und 1,5-Naphthalindisulfonsäure.

Säureadditionssalze für Fungizide sind beispielsweise die Hydrochloride, Bromide, Sulfate, Nitrate, Phosphate, Oxalate oder Dodecyl-benzolsulfonate. Die Wirksamkeit der Salze geht auf das Kation zurück, so daß die Wahl des Anions beliebig ist. Nichtphytotoxische Anionen werden bevorzugt. Sie werden hergestellt durch Umsetzung der Azolylmethyloxirane mit den entsprechenden Säuren. Metallkomplexe sind Verbindungen der Formel VIII

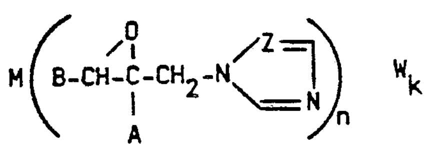

VIII,

in der A, B und X die oben angegebene Bedeutung haben und M ein Metall, z.B. Kupfer, Zink, Zinn, Mangan, Eisen, Kobalt oder Nickel bedeutet, W für das Anion einer anorganischen Säure steht, z.B. Salzsäure, Schwefelsäure, Phosphorsäure oder Bromwasserstoffsäure und n und k 1, 2, 3 oder 4 bedeuten. Sie werden hergestellt durch Umsetzung der Azolylmethoxyoxirane mit den entsprechenden Metallsalzen.

Die neuen Verbindungen der Formel I lassen sich herstellen, indem man
a) eine Verbindung der Formell II

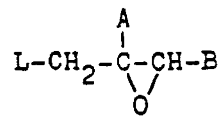

(II)

in welcher A und B die angegebenen Bedeutungen haben und L eine nukleophil substituierbare Abgangsgruppe darstellt, mit einer Verbindung der Formel III

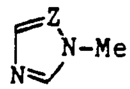

(III)

in der Me ein Wasserstoffatom oder ein Metallatom bedeutet und Z für eine CH-Gruppe oder ein Stickstoffatom steht, zur Umsetzung bringt, oder

b) eine Verbindung der Formel II, in der A und B die oben angegebenen Bedeutungen haben und L für eine Hydroxygruppe steht, mit einer Verbindung der Formel IV

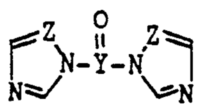

(IV),

in welcher Z die angegebene Bedeutung hat und Y ein Kohlenstoff- oder ein Schwefelatom bedeutet, zur Reaktion bringt oder

c) eine Verbindung der Formel V

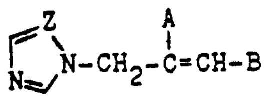

(V),

in welcher Z, A und B die angegebene Bedeutung haben, epoxidiert oder

d) eine Verbindung der Formel VI

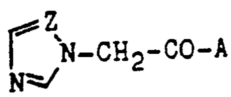

(VI),

in welcher Z und A die angegebene Bedeutung haben, mit einer Verbindung der allgemeinen Formel VII

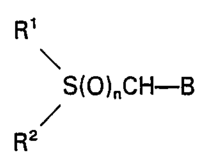

(VII),

in welcher B die angegebene Bedeutung hat, $R^1$ sowie $R^2$, die gleich oder verschieden voneinander sein können, eine Methyl- oder Phenyl-Gruppe bedeuten und n Null oder Eins bedeutet, zur Reaktion bringt und die so erhaltenen Verbindungen gegebenenfalls in ihre Salze mit physiologisch verträglichen Säuren überführt.

Die Reaktion a) erfolgt — falls Me ein Wasserstoffatom bedeutet — gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C. Zu den bevorzugten Lösungs- oder Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Geeignete Basen, die gegebenenfalls auch als säurebindende Mittel bei der Reaktion verwendet werden können, sind beispielsweise Alkalihydroxide wie Lithium-, Natrium- oder Kaliumhydroxid; Alkalicarbonate wie Natrium- oder Kaliumcarbonat oder Natrium- und Kaliumhydrogencarbonat, Überschüsse an 1,2,4-Triazol, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Benzyltri-ethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzung wird im allgemeinen bei Temperaturen zwischen 20 und 150°C, drucklos oder unter Druck, kontinuierlich oder diskontinuierlich durchgeführt.

Ist Me ein Metallatom wird die Reaktion a) gegebenenfalls in Gegenwart eines Lösungs- oder Verdünnungsmittels und gegebenenfalls unter Zusatz einer starken anorganischen oder organischen Base bei Temperaturen zwischen -10 und 120°C durchgeführt. Zu den bevorzugten Lösungs- oder Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butoxid, Lithium-, Natrium- oder Kaliumtriphenylmethyl und Naphthalin-lithium, -natrium oder -kalium.

Für die Reaktion b) kommen als Verdünnungsmittel polare organische Lösungsmittel wie Nitrile, z.B. Acetonitril, Sulfoxide, z.B. Dimethylsulfoxid, Formamide, z.B. Dimethylformamid, Ketone, z.B. Aceton, Ether, z.B. Diethylether, Tetrahydrofuran und insbesondere Chlorkohlenwasserstoffe, z.B. Methylenchlorid und Chloroform, in Frage.

Man arbeitet im allgemeinen zwischen 0 und 100°C, vorzugsweise bei 20 bis 80°C. Bei Anwesenheit eines Lösungsmittels wird zweckmäßigerweise beim Siedepunkt des jeweiligen Lösungsmittels gearbeitet.

Bei der Durchführung des Verfahrens b) setzt man auf 1 Mol der Verbindung der Formel II (L=OH), vorzugsweise etwa 1 Mol Carbonyl-bis-1,2,4-triazol-(1) bzw. Carbonyl-bis-imidazol-(1) oder 1 Mol Sulfonyl-bis-1,2,4-triazol-(1) bzw. Sulfonyl-bis-imidazol-(1) ein oder erzeugt Sulfonyl-bis-1,2,4-triazol-(1) bzw. Sulfonyl-bis-imidazol-(1) in situ. Zur Isolierung der Verbindungen der Formel I wird das Lösungsmittel abdestilliert, der Rückstand mit einem organischen Solvens aufgenommen und mit Wasser gewaschen.

Die neuen Ausgangsverbindungen II erhält man durch Epoxidierung der entsprechende Olefine IX:

$$L—CH_2—CA=CH—B \qquad (IX)$$

(vgl. G. Dittus in Houben-Weyl-Müller, Methoden der organischen Chemie, Georg Thieme Verlag, Stuttgart, 1965, Bd. VI, 3, Seite 385 ff).

Die Verbindung IX stellt man her, indem man Olefine der Formel X

$$H_3C—CA=CH—B \qquad (X)$$

nach bekannten Methoden in Allylposition halogeniert oder oxidiert.

Geeignete Halogenierungsreagenzien sind N-Chlor- und N-Bromsuccinimid in halogenierten Kohlenwasserstoffen wie Tetrachlorkohlenstoff, Trichlorethan oder Methylenchlorid bei Temperaturen zwischen 20 und 100°C. Zur Allyloxidation verwendet man Perester wie Perbenzoesäure-tert.-butylester oder Peressigsäure-tert.-butylester in Anwesenheit eines Schwermetallsalzes wie z.B. Kupfer-I-chlorid oder Kupfer-I-bromid. Man arbeitet in inerten Lösungsmitteln bei Temperaturen zwischen 10 und 100°C.

Die so erhaltenen Allyl-halogenide bzw.-alkohole IX werden anschließend in die entsprechenden Epoxide II (L=Halogen, OH) übergeführt. Dazu oxidiert man die Olefine IX mit Peroxycarbonsäuren wie Perbenzoesäure, 3-Chlorperbenzosäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff, Dichlorethan, aber gegebenenfalls auch in Essigsäure, Essigester, Aceton oder Dimethylformamid, gegebenenfalls in Gegenwart eines Puffers wie Natriumacetat, Natriumcarbonat, Natriumhydrogencarbonat, Dinatriumhydrogenphosphat, Triton B. Man arbeitet zwischen 10 und 100°C und katalysiert die Reaktion gegebenenfalls z.B. mit Iod, Natriumwolframat oder Licht. Zur Oxidation eignen sich auch alkalische Lösungen von Wasserstoffperoxid (ca. 30 %ig) in Methanol, Ethanol, Aceton oder Acetonitril bei 25 bis 30°C sowie Alkylhydroperoxide, z.B. tert.-Butylhydroperoxid, unter Zusatz eines Katalysators, z.B. Natriumwolframat, Perwolframsäure, Molybdänhexa-carbonyl oder Vanadylacetylacetonat. Die genannten Oxidationsmittel lassen sich z.T. in situ erzeugen.

Während die so erhaltenen Epoxihalogenide II (L=Halogen) gemäß Verfahren a) sofort umgesetzt werden können, läßt man die entsprechenden Epoxialkohole II (L=OH) entweder mit Verbindungen der Formel IV gemäß Verfahren b) reagieren oder überführt dieselben in reaktive Ester, die dann mit den Verbindungen III gemäß Verfahren a) umgesetzt werden.

Die Darstellung der reaktiven Ester die mit III umgesetzt werden, erfolgt nach allgemein bekannten Methoden (Houben-Weyl-Müller, Methoden der organischen Chemie, Georg Thieme Verlag, Stuttgart, 1955, Band 9, Seiten 388, 663, 671). Solche Ester sind beispielsweise Methansulfonsäureester, Trifluormethansulfonsäureester, 2,2,2-Trifluormethansulfonsäureester, Nonafluorbutansulfonsäureester, 4-Methylbenzolsulfonsäureester, 4-Brombenzolsulfonsäureester, 4-Nitrobenzolsulfonsäureester oder Benzolsulfonsäureester.

Die zum Teil unbekannten Verbindungen X lassen sich entsprechend allgemein bekannten Verfahren zur Olefinsynthese (Houben-Weyl-Müller, Methoden der organischen Chemie, Georg Thieme Verlag, Stuttgart, 1972 Bd. V, Ib) herstellen.

Das Verfahren c) erfolft analog der Epoxidierung der Verbindungen IX.

Die Ausgangsverbindungen V sind z.T. bekannt (DE—OS 25 49 798) oder lassen sich nach den dort angegebenen Methoden herstellen.

Für das erfindungsgemäße Verfahren d) setzt man literaturbekannte Azolylketone (z.B. DE—OS 20 63 857) der Formel VI mit Schwefelderivaten der Formel VII um.

Die Alkylidensulfurane VII (n = O) und Oxysulfurane VII (n = 1) werden nach bekannten allgemeinen Methoden (z.B. H.O. House, Modern Synthetic Reactions, 2nd Ed., W.A. Benjamin, Menlo Park 1972, S. 712 ff) in situ hergestellt und in inerten Lösungsmitteln, vorzugsweise Ethern wie Diethylether, Tetrahydrofuran oder Mischungen aus beiden oder in Kohlenwasserstoffen wie Pentan, Hexan oder Petrolethern bei Temperaturen zwischen −78 und 30°C mit den Azolylketonen VI umgesetzt.

Die so erhaltenen Verbindungen der Formel I werden nach üblichen Methoden isoliert, gegebenenfalls gereinigt und gegebenenfalls mit Säuren zu Salzen umgesetzt.

Überraschenderweise zeigen die erfindungsgemäßen Azol-derivate neben einer guten antibakteriellen und antimykotischen in-vitro-Wirksamkeit eine bessere, therapeutisch nutzbare in-vivo-Wirksamkeit, insbesondere gegen Dermatophyten und Candida, als bekannte Präparate. Die erfindungsgemäßen Wirkstoffe stellen somit eine wertvolle Bereicherung der Pharmazie dar.

Die Wirkung gegen Dermatophyten, Bakterien und Protozonen kann nach Methoden, wie sie beispielsweise in P. Klein, Bakteriologische Grundlagen der chemotherapeutischen Laboratoriumspraxis, Springer-Verlag Berlin, 1957, beschrieben wird, aufgezeigt werden. Die überraschende Wirkung gegenüber Hefen wurde im Pseudomyzel- und Myzelphasentest mit Candida albicans nachgewiesen (vgl. DE—OS 30 10 093). Es wurde geprüft, welche minimalen Hemmkonzentrationen (MHK-Werte) im Agardilutionstest erreicht werden. In der Tabelle 1 sind die Ergebnisse zusammengefaßt.

TABELLE 1

Antimikrobielle Wirksamkeit der neuen Verbindungen in vitro

| Substanz des Beispiels Nr. | Minimale Hemmkonzentration (MHK) µg/ml Epid. flocc. 157 | Microsp. ferrug. 5 | Trichoph. ment. 21 | Cand. alb. H | Cand. alb. M | Abs. corym. 1 | Asp. fum. 2 | Mucor pus. 6 | Staph. sur. SG 511 | Strept. faec. 8043 | E.coli muta-flor | Tri-chom. vag. | Ent-amoeb hist. |
|---|---|---|---|---|---|---|---|---|---|---|---|---|---|
| 6 | 0,125 | 0.0625 | 0,125 | 32 | >0,0039 | 8 | 0,0625 | 4 | 16 | 16 | >128 | >16 | — |
| 7 | 1 | 0,25 | 1 | 16 | 0,0156 | 1 | 4 | 2 | 16 | 16 | >128 | >16 | — |
| 9 | 1 | 0,25 | 1 | >1 | <0,125 | 4 | 16 | 2 | 128 | 128 | >128 | >16 | — |
| 1 | 0.5 | 0,125 | 0.5 | 16 | <0,0039 | 0,5 | 1 | 1 | 16 | 128 | >128 | >16 | — |
| 3 | 4 | 2 | 8 | >128 | <0,125 | 2 | >128 | 1 | 32 | >128 | >128 | >16 | >1 |
| 13 | 16 | 8 | 16 | 2 | 1 | 16 | >128 | 8 | 8 | 2 | >128 | >16 | >1 |
| 36 | 1 | 0,25 | 1 | 64 | 0,0156 | 2 | 16 | 2 | 8 | 4 | >128 | >16 | >1 |
| 18 | 2 | 2 | 2 | 128 | 0,25 | 8 | 16 | 4 | 16 | 4 | >128 | >16 | >1 |
| 19 | 8 | 2 | 8 | 64 | 2 | 16 | >128 | 8 | >128 | >128 | >128 | >16 | >1 |

Auch im Modell der Meerschweinchentrichophytie (Trichophyton mentagrophytes) vgl. Heffter-Heubner: Handbuch d. exp. Pharmakologie, Vol. XVI/IIA, sind die neuen Verbindungen bei äußerlicher Anwendung besser wirksam als Vergleichssubstanzen.

Die neuen Substanzen sind auch oral wirksam wie sich aus der Behandlung der experimentellen Candidose an der Maus zeigen läßt. Hierzu wurden Gruppen von jeweils 10 ca. 20 g schweren Mäusen 2 Tage mit je 50 mg/kg Hydrocortison i.m. vorbehandelt, um ein gutes Angehen der Infektion zu erzielen. Die Mäuse wurden dann mit je 500.000 Candida albicans-keimen i.v. infiziert und danach 4 Tage lang zweimal täglich mit je 100 mg/kg der zu prüfenden Substanzen oral befandelt.

Neben der infizierten Gruppe und der nicht behandelten Kontrollgruppe wurden zum Vergleich je eine Gruppe mit Referenzsubstanzen behandelt. Dabei zeigten die Prüfsubstanzen bessere $DC_{50}$—Werte als Referenzsubstanzen.

Im Modell der experimentellen Vaginitis mit Candida albicans an der Ratte konnten mit den Prüfsubstanzen in niedrigen therapeutischen Dosen nach oraler Gabe oder lokaler Behandlung volle Ausheilungen der Infektionen erreicht werden:

Die erfindungsgemäßen Verbindungen sind daher besonders geeignet zur oralen und äußerlichen Behandlung von Pilzinfektionen an Mensch und Tier.

Als Indikationsgebiete an Mensch und Tier sind beispielsweise zu nennen: Dermatomykosen, Dermatophytosen und Systemmykosen, insbesondere verursacht durch Dermatophyten — wie Species der Gattungen Epidermophyton, Microsporum oder Trichophyton — Hefen — wie Species der Gattungen Candida- und Schimmelpilze — wie Species der Gattungen Aspergillus, Mucor oder Absidia-.

Die Verbindungen können allein oder zusammen mit anderen bekannten Wirkstoffen, insbesondere Antibiotika, verwendet werden.

Die Herstellung der chemotherapeutischen Mittel oder Zubereitungen, die mit üblichen festen, halbfesten oder flüssigen Trägerstoffen oder Verdünnungsmitteln und den üblicherweise verwendeten pharmazeutisch-technischen Hilfsstoffen entsprechend der gewünschten Applikationsart mit einer zur Anwendung geeigneten Dosierung erfolgt in üblicher Weise, insbesondere durch Vermischen (vgl. H. Sucker et al, Pharmazeutische Technologie, Thieme-Verlag, Stuttgart, 1978).

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions etc. in Betracht.

Die therapeutisch wirksame Verbindung ist in pharmazeutischen Zubereitungen vorzugsweise in einer Konzentration von 0,5 bis 90 Gewichtsprozent der Gesamtmischung vorhanden.

Im allgemeinen können bei oraler Verabfolgung sowohl in der Human- als auch in der Veterinärmedizin der oder die Wirkstoffe in Mengen von etwa 1,0 bis etwa 10,0, vorzugsweise 2 bis 6 mg/kg Körpergewicht pro Tag, vorzugsweise in Form mehrerer Einzelgaben zur Erzielung der gewünschten Ergebnisse verabreicht werden. Es kann jedoch erforderlich sein, von den genannten Dosierungen abzuweichen, und zwar in Abhängigkeit von der Art und der Schwere der Erkrankung, der Art der Zubereitung und der Applikation des Arzneimittels, sowie dem Zeitraum bzw. Intervall, innerhalb welchem die Verabreichung erfolgt. So kann es in einigen Fällen ausreichend sein, mit weniger als der oben genannten Menge Wirkstoffe auszukommen, während in anderen Fällen die oben angeführte Wirkstoffmenge überschritten werden muß.

Die neuen Verbindungen wirken nicht nur gegen humanpathogene Pilze sondern können auch als Fungizide in der Landwirtschaft eingesetzt werden.

Sie zeichnen sich hier durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz verwendet werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Bananen, Erdnüsse, Zuckerrohr, Obst und Zierpflanzen im Gartenbau, sowie Gemüse — wie Gurken, Bohnen und Kürbisgewächse —.

Die neuen Verbindungen sind insbesondere geeignet zur Bekämpfung folgender Pflanzenkrankheiten:

Erysiphe graminis (echter Mehltau) in Getreide, Erysiphe cichoracearum (echter Mehltau) an Kürbisgewächsen, Podosphaera leucotricha an Äpfeln, Uncinula necator an Reben, Erysiphe polygoni an Bohnen, Sphaerotheca pannosa an Rosen, Puccinia-Arten an Getreide, Rhizoctonia solani an Baumwolle sowie Helminthosphoriumarten and Getreide, Ustilago-Arten an Getreide und Zuckerrohr, Rhynchosporium secale an Getreide, Venturia inaequalis (Apfelschorf), Botrytis cinerea an Erdbeeren und Reben, Septoria nodorum an Getreide.

Die Verbindungen werden angewendet, indem man die Pflanzen mit den Wirkstoffen besprüht oder bestäubt oder die Samen der Plfanzen mit den Wirkstoffen behandelt. Die Anwendung erfolgt vor oder nach der Infektion der Pflanzen oder Samen durch die Pilze.

Folgende holz- und anstrichverfärbende Pilze, Moderfäulepilze und holzzerstörende Pilze lassen sich beispielsweise mit den erfindungsgemäßen Mitteln bekämpfen: Aureobasidium pullulans, Sclerophoma pityophila, Ceratocystis spec., Paecilomyces variotii, Hormiscium spec., Stemphylium spec., Fhoma violacea, Cladosporium herbarum, Trichoderma viride, Chaetomium globosum, Humicola grisea, Merulius

lacrimans, coniophora puteana, Lentinus lepideus, Lenzites trabea, Trametes versicolor, Stereum hirsutum, Fomes annosus.

Die neuen Substanzen können in die überlichen Fomulierungen übegeführt werden, wie Lösungen, Emulsionen, Suspensionen, Stäube, Pulver, Pasten und Granulate. Die Anwendungsformen richten sich ganz nach den Verwendungszwecken; sie sollten in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel die nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha. Die neuen Verbindungen können auch in Materialschutz eingesetzt werden. Bei der Anwendung der Wirkstoffe im Materialschutz, z.B. als Fungizide für Anstrichfarben und Weich-Polyvinylchlorid, betragen die Aufwandmengen 0,05 bis 5% (Gew.%) Wirkstoff, bezogen auf das Gesamtgewicht der zu konservierenden Farben bzw. des mikrozid auszurüstenden Polyvinylchlorids. Die neuen Wirkstoffe können für den Holzchutz in Zubereitungen, wie Lösungen, Emulsionen, Pasten und Öldispersionen, angewendet werden. Die Zubereitungen enthalten im allgemeinen zwischen 0,1 und 95 Gew.% Wirkstoff, vorzugsweise 0,25 bis 50%. Die Aufwandmengen betragen je nach Art des gewünschten Effektes 0,5 bis 8 g Wirkstoff je $m^2$ zu schützender Holzoberfläche bzw. 50 bis 4000 g Wirkstoff/$m^3$ Holz. Anstrichfarben enthalten 1,5 bis 2 Gew.% Wirkstoff. Zum Schutz von Holzwerkstoffen werden die Wirkstoffe als Emulsion oder im Untermischverfahren dem Klebstoff im Mengen von 2 bis 6 Gew.% zugesetzt.

Die Anwendung der Wirkstoffe erfolgt durch Streichen, Spritzen, Sprühen, Tauchen oder Druckimprägnierungs- oder Diffusionsverfahren.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen; Vernebeln, Verstäuben, Verstreuen, Beizen oder Gießen.

Die erfindungsgemäßen Mittel können in diesen Anwendungsformen auch zusammen mit anderen Wirkstoffen vorliegen, wie z.B. Herbiziden, Insektiziden, Wachstrumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,

Dithiocarbamate und deren Derivate, wie
Ferridimethyldithiocarbamat,
Zinkdimethyldithiocarbamat,
Mangan-Zink-ethylendiamin-bis-dithiocarbamat und
Zinkethylenbisdithiocarbamat,
Tetramethylthiuramdisulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und N,N'-Polypropylen-bis-(thiocarbamoyl)-disulfid;

Nitroderivate, wie
Dinitro-(1-methylheptyl)-phenylcrotonat,
2-sec.-Butyl-4,6-dinitrophenyl-3,3-dimethylacrylat,
2-sec.-Butyl-4,6-dinitrophenyl-isopropylcarbonat;

heterocyclische Substanzen, wie
N-(1,1,2,2-Tetrachlorethylthio)-tetrahydrophthalimid,
N-Trichlormethylthio-tetrahydrophthalimid,
2-Heptadecyl-2-imidazolin-acetat
2,4-Dichlor-6-(o-chloranilino)-s-triazin

O,O-Diethyl-phthalimidophosphonothioat
5-Amino-1-(bis-(dimethylamino)-phosphinyl)-3-phenyl-1,2,4-triazol)
2,3-Dicyano-1,4-Dithioaanthrachinon
2-Thio-1,3-dithio-(4,5-b)-chinoxalin
1-(Butylcarbamoyl)-2-benzimidazol-carbaminsäuremethylester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carbonanilido-6-methyl-1,4-oxathiin
2-(Furyl-(2))-benzimidazol
Piperazin-1,4-diylbis-(1-(2,2,2-trichlor-ethyl)-formamid
2-(Thiazolyl-(4)-benzimidazol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-glutarimid
Hexachlorbenzol
N-Dichlorfluormethylthio-N',N'-dimethyl-N-phenyl-schwefelsäurediamid
2,5-Dimethyl-furan-3-carbonsäureanilid
2-Methyl-benzoesäure-anilid
2-Jod-benzoesäure-anilid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,

1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazol-yl-harnstoff,
N-Cyclohexyl-N-methoxy-2,5-dimethyl-furan-3-carbonsäureamid,

2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-3,4-dioxo-1,3-oxazolidin,
N-[3-(p-tert.-Butylphenyl)-2-methyl-propyl]-cis-2,6-dimethylmorpholin.

N-Formyl-N-morpholin-2,2,2-trichlorethylacetal
1-[2-(2,4-Dichlorphenyl)-4-ethyl-1,3-dioxolan-2-yl-methyl]-2H-1,2,4-triazol
1-[2-(2,4-Dichlorphenyl)-4-n-propyl-1,3-dioxolan-2-yl-methyl]-1H-1,2,4-triazol.

Organozinnverbindungen, wie Tributylzinnoxid und Tributylzinnbenzoat
Methylenbisthiocyanat
Alkyl-dimethyl-benzylammoniumchlorid
Cetyl-pyridiniumchlorid
Chlorierte Phenole, wie Tetra- und Pentachlorphenol
Tetrachlorisophthalsäure-dinitril
2-Halogenbenzoesäureanilid
N-Cyclohexyl-N-methoxy-2,5-dimethyl-furan-3-carbonsäureamid
N,N-Dimethyl-N'-phenyl-(N-fluormethylthio)-sulfamid
N-Phenyl-N,N'-dimethyl-N'-fluordichlormethyl-thiosulfonyl-diamid
Benzimidazol-2-carbaminsäure-methylester
2-Thiocyanomethyl-thiobenzothiazol
Kupfernaphthenat
Kupfer-6-oxychinolin
Alkali- und Metallsalze des N'-Hydroxy-N-cyclohexyl-diazeniumoxide

Für die folgenden Versuche wurden zu Vergleichszwecken die bekannten Wirkstoffe
1-(2,4-Dichlorphenyl)-2-(imidazol-1-yl)-ethan-1-ol A (FR—22 49 616) und
(2,4-Dichlorphenyl)-1,2,4-triazol-1-yl-methylketon B (DE—OS 24 31 407)
verwendet.

Versuch 1

Wirksamkeit gegen Weizenmehltau

Blätter von in Töpfen gewachsenen Weizenkeimlingen der Sorte "Jubilar" wurden mit wäßriger Spriztbrühe, die 80% (Gew.%) Wirkstoff und 20% Emulgiermittel in der Trockensubstanz enthielt, besprüht und 24 Stunden nach dem Antrocknen des Spritzbelages mit Oidien (Sporen) des Weizenmehltaus (erysiphe graminis var. tritici) bestäubt. Die Versuchspflanzen wurden anschließend im Gewächshaus bei Temperaturen zwischen 20 und 22°C und 75 bis 80% relativer Luftfeuchtigkeit aufgestellt. Nach 7 Tagen wurde das Ausmaß der Mehltauentwicklung ermittelt.

Das Ergebnis des Versuches zeigte, daß beispielsweise die Verbindungen 1, 13, 20, 21, 22, 23, 24, 25, 31, 33 und 35 bei Anwendung als 0,025; 0,006; 0,0015 %igen Spritzbrühen eine bessere fungizide Wirkung hatten (beispielsweise 100 %ige Wirkung) als die Wirkstoffe A oder B (beispielsweise 90 %ige Wirkung).

Versuch 2

Wirksamkeit gegen Weizenbraunrost

Blätter von in Töpfen gewachsenen Weizensämlingen der Sorte "Jubilar" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95%) gestellt. Während dieser Zeit keimten die Sporen aus und die Keimschläuche drangen in das Blattgewebe ein. Die infizierten Pflanzen wurden anschließend mit wäßrigen Spritzbrühen, die 80% Wirkstoff und 20% Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70% relativer Luftfeuchte aufgestellt. Nach 8 Tagen wurde das Ausmaß der Rostpilzentwicklung auf den Blättern ermittelt.

Das Ergebnis des Versuches zeigte, daß die Verbindungen 3, 4, 6, 10, 19, 20, 21, 22, 23, 27, 30, 31, 32, 33 und 35 bei Anwendung als 0,025; 0,006; 0,0015 %ige Spritzbrühen eine bessere fungizide Wirkung (beispielsweise 100 %ige Wirkung) hatten als die Wirkstoffe A oder B (beispielsweise 50 %ige Wirkung).

Versuch 3

Wirksamkeit gegen Gurkenmehltau

Blätter von in Töpfen gewachsenen Gurkenkeimlingen der Sorte "Chinesische Schlange" wurden im Zweiblattstadium mit einer Sporensuspension des Gurkenmehltaus (Erysiphe cichoracearum) besprüht. Nach etwa 20 Stunden wurden die Versuchspflanzen mit wäßriger Spritzbrühe, die 80% Wirkstoff und 20% Emulgiermittel in der Trockensubstanz enthielt, bis zur Tropfnässe besprüht. Nach dem Antrocknen des Spritzbelages wurden sie anschließend im Gewächshaus bei Temperaturen zwischen 20 und.22°C und 70 bis 80%.relativer Luftfeuchtigkeit aufgestellt. Zur Beurteilung der Wirksamkeit der neuen Stoffe wurde das Ausmaß der Pilzentwicklung nach 21 Tagen beurteilt.

Das Ergebnis des Versuches zwigt, daß beispielsweise die Verbindungen 1, 4, 6, 10, 11, 17, 20, 21, 22, 23, 25, 27, 29, 30, 31, 32, 33 und 34 bei Anwendung als 0,025 %ige Spritzbrühe eine gute fungizide Wirkung hatten (beispielsweise 100 %ige Wirkung).

Versuch 4

Wirksamkeit gegen Botrytis cinerea an Paprika

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20% Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cenerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt, daß die entsandenen Blattnekrosen den überwiegenden Teil der Blätter bedeckten.

Das Ergebnis des Versuches zeigte, daß beispielsweise die Verbindungen 1, 3, 4, 6, 10, 11, 19, 20, 21, 22, 24, 25, 26, 27, 28, 29, 31, 33 und 35 bei Anwendung als 0,05 %ige Spritzbrühe eine bessere fungizide Wirkung hatten (beispielsweise 97 %ige Wirkung) als die Wirkstoffe A oder B (beispielsweise 70 %ige Wirkung).

Versuch 5

Filtrierpapierscheiben mit einem Durchmesser von 13 mm und einer Stärke von 1 mm werden mit 0,2 ml Lösungen getränkt, die jeweils 200 Teile Wirkstoff je Million Teile Lösung (ppm) enthalten. Die Scheiben werden dann auf einem 2 %igen Malzextraktagar in Petrischalen gelegt, die zuvor getrennt mit Sporen des holzverfärbenden Pilzes Pullularia pullalans beimpft wurden. Anschließend werden die Schalen 3 Tage lang bei 22 bis 24°C bebrütet. Nach dieser Zeit hat sich der Pilz in den Kontrollschalen sehr gut entwickelt.

Die fungizide Wirksamkeit der Wirkstoffe wird anhand der um die Filtrierpapierscheiben herum entstandenen pilzfreien Zonen (Hemmhöfe); wie folgt beurteilt:

– kein Hemmhof (keine fungizide Wirksamkeit)
\+ kleiner Hemmhof 2 mm (geringe fungizide Wirksamkeit)
++ mittlerer Hemmhof 2 bis 6 mm (gute fungizide Wirksamkeit)
+++ großer Hemmhof 6 mm (sehr gute fungizide Wirksamkeit)

| Wirkstoff Nr. | Wirksamkeit gegen Pullularia pullalans |
|---|---|
| 31 | +++ |
| 39 | +++ |
| Kontrolle | – |

Versuch 6

Die Wirkstoffe werden in Aceton gelöst, in Mengen von 40 ppm einem verflüssigten 5 %igen Malzextraktagar zugesetzt. Der Agar wird in Petrischalen ausgegossen und nach dem Erstarren werden die fungizidhaltigen Nähragarplatten zentral mit Myzel der holzzerstörenden Pilze Coniophora puteana und Trametes versicolor, des Moderfäule und Stockflecken verursachenden Pilzes Chaetomium globosum sowie mit Sporen des grünen Holzschimmels Trichoderma viride beimpft.

Nach fünftägiger Bebrütung der Schalen bei 25°C wird die Entwicklung der Pilzkolonien auf dem Nährboden im Vergleich mit der Kontrolle (ohne Wirkstoff-Zusatz) beurteilt:

0 = kein Pilzwachstum (Pilzmyzel abgetötet)
1 = geringes Pilzwachstum (bis 1/3 der Agaroberfläche bewachsen)
3 = mittleres Pilzwachstum (bis 2/3 der Agaroberfläche bewachsen)
5 = ungehemmtes Pilzwachstum (gesamte Agaroberfläche bewachsen).

| Wirkstoff Nr. | Wirksamkeit gegen ... | | | |
|---|---|---|---|---|
| | Coniophora puteana | Trametes versicolor | Chaetomium globosum | Trichoderma viride |
| 10 | 0 | 0 | 0 | 0 |
| 31 | 0 | 0 | 0 | 0 |
| 33 | 0 | 0 | 1 | 0 |
| 34 | 0 | 0 | 1 | 0 |
| 35 | 0 | 0 | 0 | 0 |
| 37 | 0 | 0 | 0 | 0 |
| 38 | 0 | 0 | 1 | 0 |
| 39 | 0 | 0 | 0 | 0 |
| Kontrolle (ohne Wirkstoff) | 5 | 5 | 5 | 5 |

Die folgenden Beispiele erläutern die Erfindung.

I. Herstellung der Ausgangsstoffe

Beispiel A

In eine Lösung von 229 g 2,4-Dichlorbenzyltriphenylphosphoniumchlorid un 800 ml trockenem Methanol wurden bei 10°C 63,6 g Kalium-tert.-butylat in 300 ml trockenem Methanol eingetragen und nach einer halben Stunde 77,2 g 4-Chloracetophenon zugegeben. Die Reaktionslösung wurde 3 Stunden unter Rückfluß gekocht, dann bei Raumtemperatur das abgeschiedene Salz abfiltriert und das Filtrat im Vakuum eingedampft. Durch Digerieren des Rückstandes mit Petrolether (50 bis 70°C) wurde vom Triphenylphosphinoxid abgetrennt und die Lösung im Vakuum eingedampft.

Der Rückstand wurde in 1 l Tetrachlorkohlenstoff aufgenommen und mit 81,7 g N-Bromsuccinimid und 4 g 2,2'-Azoisobuttersäuredinitril unter Rückfluß gekocht. Nach beendeter Reaktion wurde das Succinimid durch Filtration abgetrennt, das Filtrat im Vakuum eingedampft und der Rückstand aus Methanol umkristallisiert. Man erheilt 73,4 g (38,8%) Z-1-(2,4-Dichlorphenyl)-2-(4-chlorphenyl)-3-brom-propen-1, Fp = 128°C.

Beispiel B

Zu 14,6 g Magnesiumspänen in 400 ml trockenem Diethylether wurden 118 g 2,4-Dichlorbenzylchlorid bei Siedetemperatur zugetropft. Nach Beendigung der Reaktion gab man 77,3 g 4-Chloracetophenon in 400 ml trockenem Diethylether hinzu. Anschließend zersetzte man mit wäßriger Ammoniumchlorid-Lösung, trennte die organische Phase ab, wusch sie neutral und trocknete über Natriumsulfat. Nach dem Einengen im Vakuum wurde der Rückstand in 1 l Toluol aufgenommen und mit 4 g 4-Methylbenzolsulfonsäure am Wasserabscheider zum Rückfluß erhitzt. Nach beendeter Dehydratisierung, wurde die Toluol-Phase mit Natriumcarbonat-Lösung und Wasser gewaschen, über Natriumsulfat getrocknet und das Lösungsmittel abgedampft. Der Rückstand lieferte aus Methanol 107 g (71,9%) E-1-(2,4-Dichlorphenyl)-2-(4-chlorphenyl)-propen-1, Fp = 84 bis 85°C.

Beispiel C

104 g E-1-(2,4-Dichlorphenyl)-2-(4-chlorphenyl)-propen-1 wurden mit 62,3 g N-Bromsuccinimid und 5 g 2,2'-Azoisobuttersäuredinitril in 1 l Tetrachlorkohlenstoff unter Rückfluß gekocht, das ausgeschiedene Succinimid abfiltriert und das Filtrat im Vakuum eingedampft.

Behandeln des Rückstandes mit Methanol ergab 91,5 g (69,4%) Z-1-(2,4-Dichlorphenyl)-2-(4-chlorphenyl)-3-brom-propen-1, Fp = 128°C.

Beispiel D

58,9 g Z-1-(2,4-Dichlorphenyl)-2-(4-chlorphenyl)-3-brom-propen-1 (vgl. Beispiel A) wurden mit 52,3 g 3-Chlorperoxybenzoesäure in 590 ml Chloroform unter Rückfluß gekocht. Nach beendeter Reaktion wurde die Chloroform-Phase mit wäßriger Natriumhydrogencarbonat-Lösung und Wasser säurefrei gewaschen, über Natriumsulfat getrocknet und im Vakuum eingedampft. Aus dem Rückstand erhielt man mit Methanol zwei Kristallfraktionen:

1. 41,3 g (70,2%) 2-Brommethyl-2-(4-chlorphenyl)-3-(2,4-dichlorphenyl)-oxiran (Isomer A), Fp = 98 bis 99°C und
2. 12 g (20,4%) 2-Brommethyl-2-(4-chlorphenyl)-3-(2,4-dichlorphenyl)-oxiran (Isomer B), Fp = 93 bis 95°C.

II. Herstellung der Endprodukte

Beispiel 1

Eine Lösung von 10 g 2-Brommethyl-2-(4-chlorphenyl)-3-(2,4-dichlorphenyl)-oxiran (Isomer A) (vgl. Beispiel D) in 50 ml N,N-Dimethylformamid wurde bei 100°C zu einer Schmelze aus 15,6 g Imidazol mit 1,37 g Natriummethylat, aus der man das freigesetzte Methanol zuvor abdestillieren ließ, zugetropft. Nach 8 Stunden wurde die Reaktionslösung auf Wasser gegeben und mit Essigsäureethylester extrahiert; die organische Phase wurde mit Wasser gewaschen, über Natriumsulfat getrocknet und im Vakuum eingedampft. Der Rückstand wurde an einer Kieselgelsäule mit Methylenchlorid/Methanol (100:2) chromatographiert. Die gereinigten Fraktionen wurden eingedampft und aus Diisopropylether kristallisiert. Man erheilt 4,6 g (47,5%) 2-(1H-Imidazol-1-yl-methyl)-2-(4-chlorphenyl)-3-(2,4-dichlorphenyl)-oxiran (Isomer A), Fp = 102 bis 103°C.

Beispiel 2

6,2 g Imidazol und 1,3 g Natriumhydrid (50 %ige Dispersion in Mineralöl) wurden in 50 ml N,N-Dimethylformid vorgelegt und bei Raumtemperatur mit einer Lösung aus 12 g 2-Brommethyl-2-(4-chlorphenyl)-3-(2,4-dichlorphenyl)-oxiran (Isomer B) (vgl. Beispiel D) und 5 g Kaliumiodid in 50 ml N,N-Dimethylformamid versetzt. Nach 8 h wurde die Reaktionslösung auf Wasser gegeben und mit Essigsäureethylester extrahiert. Die organische Phase wurde mit Wasser gewaschen, über Natriumsulfat getrocknet und das Lösungsmittel im Vakuum abgedampft.

Der Rückstand wurde aus Diisopropylether umkristallisiert und lieferte 9,4 g (82,5%) 2-(1H-Imidazol-1-yl-methyl)-2-(4-chlorphenyl)-3-(2,4-dichlorphenyl)-oxiran (Isomer B), Fp = 109°C.

Beispiel 3

20,9 g 1,2,4-Triazol und 4,4 g Natriumhydrid (50 %ige Dispersion in Mineralöl) wurden in 150 ml N,N-Dimethylformamid vorgelegt und bei Raumtemperatur mit einer Lösung aus 39,2 g 2-Brommethyl-2-(4-chlorphenyl)-3-(2,4-dichlorphenyl)-oxiran (Isomer A) (vgl. Beispiel D) und 16,6 g Kaliumiodid in 150 ml N,N-Dimethylformamid versetzt. Nach 8 h wurde wie in Beispiel 2 aufgearbeitet und man erhielt aus Diisopropylether 31 g (81,9%) 2-(1,2,4-Triazol-1-yl-methyl)-2-(4-chlorphenyl)-3-(2,4-dichlorphenyl)-oxiran (Isomer A), Fp = 119°C.

Entsprechend Beispiel 1 und 2 wurden bzw. können die in Tabelle I aufgeführten Verbindungen hergestellt werden.

TABELLE

| Beispiel Nr. | A | B | Z | Diastereomer | Fp.[°C] |
|---|---|---|---|---|---|
| 4 | 4-Br—$C_6H_4$ | $C_6H_5$ | CH | A | 152—153 |
| 5 | 4-Br—$C_6H_4$ | 4-Cl—$C_6H_4$ | CH | A | 143—144 |
| 6 | $C_6H_5$ | 2,4-$Cl_2$—$C_6H_3$ | CH | A | 103 |
| 7 | 4-Br—$C_6H_4$ | 2,4-$Cl_2$—$C_6H_3$ | CH | A | 107—108 |
| 8 | 2,4-$Cl_2$—$C_6H_3$ | 4-Cl—$C_6H_4$ | CH | A | 135 |
| 9 | 4-Cl—$C_6H_4$ | $C_6H_5$ | CH | A | 138 |
| 10 | 4-Br—$C_6H_4$ | 2,4-$Cl_2$—$C_6H_3$ | N | A | 133—134 |
| 11 | 4-Cl—$C_6H_4$ | 4-$C(CH_3)_3$—$C_6H_4$ | CH | B | 160—162 |
| 12 | 4-Cl—$C_6H_4$ | 4-$C(CH_3)_3$—$C_6H_4$ | N | A | 176—177 |
| 13 | 2,4-$Cl_2$—$C_6H_3$ | 4—$C(CH_3)_3$—$C_6G_4$ | CH | A | 132—134 |
| 14 | 4-$C(CH_3)_3$—$C_6H_4$ | 4-Cl—$C_6H_4$ | CH | A | 100—152 |
| 15 | 4-$C(CH_3)_3$—$C_6H_4$ | 4-Cl—$C_6H_4$ | N | A | 105—107 |
| 16 | 4-$C(CH_3)_3$—$C_6H_4$ | 2,4-$Cl_2$—$C_6H_3$ | CH | A | 101—113 |
| 17 | 4-$C(CH_3)_3$—$C_6H_4$ | 2,4-$Cl_2$—$C_6H_3$ | N | A | 108—111 |
| 18 | 4-Cl—$C_6H_4$ | 3-$OCH_3$—$C_6H_4$ | CH | A×HCl | 173 |
| 19 | 4-Cl—$C_6H_4$ | 3-$OCH_3$—$C_6H_4$ | N | A | 77 |
| 20 | $C_6H_5$ | 2,4-$Cl_2$—$C_6H_3$ | N | A | 159—161 |
| 21 | 4-Cl—$C_6H_4$ | 3-$CF_3$—$C_6H_4$ | CH | A | 101—104 |
| 22 | 4-Cl—$C_6H_4$ | 3-$CF_3$—$C_6H_4$ | N | A | 107—109 |
| 23 | $C_6H_5$ | 3-$CF_3$—$C_6H_4$ | CH | A | 77—78,5 |
| 24 | $C_6H_5$ | 3-$CF_3$—$C_6H_4$ | N | A×HCl | 131—133 |
| 25 | $C_6H_5$ | $C_6H_5$ | CH | A | 108—110 |
| 26 | 4-Cl—$C_6H_4$ | 4-F—$C_6H_4$ | CH | A | 103—132 |
| 27 | $C_6H_5$. | 4-Cl—$C_6H_4$ | CH | A | 105—106 |
| 28 | $C_6H_5$ | $C_6H_5$ | N | A | 116—118 |
| 29 | $C_6H_5$ | 4-Cl—$C_6H_4$ | N | A | 114—115 |
| 30 | 4-Cl—$C_6H_4$ | $C_6H_5$ | N | A | 106—110 |
| 31 | 4-$C_6H_5$—$C_6H_4$ | 4-Cl—$C_6H_4$ | N | A | 163—165 |
| 32 | 4-Br—$C_6H_4$ | $C_6H_5$ | N | A | 115—120 |
| 33 | 4-Br—$C_6H_4$ | 4-Cl—$C_6H_4$ | N | A | 115—120 |
| 34 | 4-Cl—$C_6H_4$ | 4-F—$C_6H_4$ | N | A | 112—117 |

TABELLE (fortsetzung)

| Beispiel Nr. | A | B | Z | Diastereomer | Fp.[°C] |
|---|---|---|---|---|---|
| 35 | 4-Cl—$C_6H_4$ | 4-Br—$C_6H_4$ | N | A | 115—119 |
| 36 | 4-Cl—$C_6H_4$ | 4-Br—$C_6H_4$ | CH | A | 114—116 |
| 37 | 4-Cl—$C_6H_4$ | 4-Br—$C_6H_4$ | CH | B | 179—181 |
| 38 | 2,4-$Cl_2$—$C_6H_3$ | 4-Br—$C_6H_4$ | CH | A | 135—139 |
| 39 | 4-Cl—$C_6H_4$ | 4-F—$C_6H_4$ | N | B | 219—223 |
| 40 | 4-Cl—$C_6H_4$ | 4-Br—$C_6H_4$ | N | B | 210—213 |
| 41 | 2,4-$Cl_2$—$C_6H_3$ | 4-Br—$C_6H_4$ | N | A | 108—110 |
| 42 | 2,4-$Cl_2$—$C_6H_3$ | $C_6H_5$ | CH | A | Harz |
| 43 | 2,4-$Cl_2$—$C_6H_3$ | $C_6H_5$ | CH | B | 118—121 |
| 44 | 2,4-$Cl_2$—$C_6H_3$ | $C_6H_5$ | N | A | Harz |
| 45 | 2,4-$Cl_2$—$C_6H_3$ | $C_6H_5$ | N | B | Harz |
| 46 | 4-($SO_2$—$C_6H_5$)—$C_6H_4$ | 4-Cl—$C_6H_4$ | CH | A | 193—195 |
| 47 | 4-($SO_2$—$C_6H_5$)—$C_6H_4$ | 4-Cl—$C_6H_4$ | CH | B | 204—205 |
| 48 | 4-($SO_2$—$C_6H_5$)—$C_6H_4$ | 4-Cl—$C_6H_4$ | N | A | 132—135 |
| 49 | 4-($SO_2$—$C_6H_5$)—$C_6H_4$ | 4-Cl—$C_6H_4$ | N | B | 175—177 |
| 50 | 2,4-$Cl_2$—$C_6H_3$ | 4-Cl—$C_6H_4$ | N | A | |
| 51 | 4-$C_6H_5$—$C_6H_4$ | 4-Cl—$C_6H_4$ | CH | | |
| 52 | 2-Cl—$C_6H_4$ | 4-Cl—$C_6H_4$ | CH | | |
| 53 | 2-Cl—$C_6H_4$ | 4-Cl—$C_6H_4$ | N | | |
| 54 | 4-Cl—$C_6H_4$ | 3-Cl—$C_6H_4$ | CH | | |
| 55 | 4-Cl—$C_6H_4$ | 3-Cl—$C_6H_4$ | N | | |
| 56 | $C_6H_5$ | 3,4-$Cl_2$—$C_6H_3$ | CH | | |
| 57 | $C_6H_5$ | 3,4-$Cl_2$—$C_6H_3$ | N | | |
| 58 | 3,5-$Cl_2$—$C_6H_3$ | 4-Cl—$C_6H_4$ | CH | | |
| 59 | 3,5-$Cl_2$—$C_6H_3$ | 4-Cl—$C_6H_4$ | N | | |
| 60 | 2-$OCH_3$—$C_6H_4$ | 2,4-$Cl_2$—$C_6H_3$ | CH | | |
| 61 | 2-$OCH_3$—$C_6H_4$ | 2,4-$Cl_2$—$C_6H_3$ | N | | |
| 62 | 3,4-(O—$CH_2$—O)—$C_6H_3$ | 4-Br—$C_6H_4$ | CH | | |
| 63 | 3,4-(O—$CH_2$—O)—$C_6H_3$ | 4-Br—$C_6H_4$ | N | | |
| 64 | 4-O—$C(CH_3)_3$—$C_6H_4$ | 2,4-$Cl_2$—$C_6H_3$ | CH | | |

TABELLE (fortsetzung)

| Beispiel Nr. | A | B | Z | Diastereomer | Fp.[°C] |
|---|---|---|---|---|---|
| 65 | 4-O—$C(CH_3)_3$—$C_6H_4$ | 2,4-$Cl_2$—$C_6H_3$ | N | | |
| 66 | 4-$CH_3$—$C_6H_4$ | $C_6H_5$ | CH | | |
| 67 | 4-$CH_3$—$C_6H_4$ | $C_6H_5$ | N | | |
| 68 | 4-(O—$C_6H_5$)—$C_6H_4$ | 4-Br—$C_6H_4$ | CH | | |
| 69 | 4-(O—$C_6H_5$)—$C_6H_4$ | 4-Br—$C_6H_4$ | N | | |
| 70 | $C_6H_5$ | 4-$NO_2$—$C_6H_4$ | CH | | |
| 71 | $C_6H_5$ | 4-$NO_2$—$C_6H_4$ | N | | |
| 72 | 2-$C_{10}H_7$ | 2,4-$Cl_2$—$C_6H_3$ | CH | A | 135 |
| 73 | 2-$C_{10}H_7$ | 2,4-$Cl_2$—$C_6H_3$ | N | A | 151 |
| 74 | 4-Cl—$C_6H_4$ | 1-$C_{10}H_7$ | CH | | |
| 75 | 4-Cl—$C_6H_4$ | 1-$C_{10}H_7$ | N | | |
| 76 | 4-Cl—$C_6H_4$ | 4-Cl—$C_6H_4$ | CH | A | 115—120 |
| 77 | 4-Cl—$C_6H_4$ | 4-Cl—$C_6H_4$ | N | A | 105—108 |
| 78 | 2-$C_{10}H_7$ | 4-Cl—$C_6H_4$ | CH | A | 140 |
| 79 | 2-$C_{10}H_7$ | 4-Cl—$C_6H_4$ | N | A | 107 |
| 80 | 2,4-$Cl_2$—$C_6H_3$ | 4-$C(CH_3)_3$—$C_6H_4$ | CH | B | 142—143 |
| 81 | 3,4-$Cl_2$—$C_6H_3$ | 4-Br—$C_6H_4$ | N | A | 130—134 |
| 82 | 4-Br—$C_6H_4$ | $C_6H_5$ | CH | A | 198—200 |
| | (Salz mit 1/2 Mol $CuCl_2$) | | | | |

Beispiele für pharmazeutische Zubereitungen:

## Beispiel A

Tablette mit 250 mg Wirkstoff

Zusammensetzung für 1000 Tabletten:

| | |
|---|---|
| Wirkstoff des Beispiels Nr. 3 | 250 g |
| Kartoffelstärke | 100 g |
| Milchzucker | 50 g |
| Gelatinelösung 4% | 45 g |
| Talkum | 10 g |

Herstellung:

Der fein gepulverte Wirkstoff, Kartoffelstärke und Milchzucker werden gemischt. Die Mischung wird mit ca. 45 g 4% Gelatinelösung durchfeuchtet, feinkörnig granuliert und getrocknet. Das trockene Granulat wird gesiebt, mit 10 g Talkum vermischt und auf einer Rundläufer-Tablettier-maschine zu Tabletten verpreßt. Die Tabletten werden in dicht schließende Behälter aus Polypropylen gefüllt.

## Beispiel B

Creme aus 1% Wirkstoff

| | | |
|---|---|---|
| Wirkstoff des Beispiels Nr. 3 | | 1,0 g |
| Glycerinmonostearat | | 10,0 g |
| Cetylalkohol | | 4,0 g |
| Polyethylenglykol-400-stearat | | 10,0 g |
| Polyethylenglykol-sorbitanmonostearat | | 10,0 g |
| Propylenglykol | | 6,0 g |
| p-Hydroxybenzoesäuremethylester | | 0,2 g |
| Entmineralisiertes Wasser | ad | 100,0 g |

Herstellung:

Der feinst gepulverte Wirkstoff wird mit Propylenglykol suspendiert und die Suspension in die auf 65°C erwärmte Schmelze aus Glycerinmonostearat, Cetylalkohol, Polyethylenglykol-400-stearat und Polyethylenglykolsorbitan-monostearat gerührt. In diese Mischung wird die 70°C heiße Lösung des p-Hydroxybenzoesäuremethylesters in Wasser emulgiert. Nach dem Erkalten wird die Creme über eine Kolloidmühle homogenisiert und in Tuben abgefüllt.

## Beispiel C

Puder mit 1% Wirkstoff

| | |
|---|---|
| Wirkstoff des Beispiels Nr. 3 | 1,0 g |
| Zinkoxid | 10,0 g |
| Magnesiumoxid | 10,0 g |
| Hochdisperses Siliciumdioxid | 2,5 g |
| Magnesiumstearat | 1,0 g |
| Talkum | 74,5 g |

Herstellung:

Der Wirkstoff wird auf einer Luftstrahlmühle mikronisiert und mit den anderen Bestandteilen homogen vermischt. Die Mischung wird durch ein Sieb (Maschenweite Nr. 7) geschlagen und in Polyethylenbehälter mit Streueinsatz abgefüllt.

Beispiele für Fungizid-Zubereitungen:

## Beispiel a

Man vermischt 90 Gewichtsteile der Verbindung des Beispiels 5 mit 10 Gewichtsteilen N-Methyl-alphapyrrolidon und erhält eine Lösung, die zur Anwendung in Form kleinster Tropfen geeignet ist.

## Beispiel b

10 Gewichtsteile der Verbindung des Beispiels 7 werden in einer Mischung gelöst, die aus 90 Gewichtsteilen Xylol, 6 Gewichtsteilen des Anlagerungsproduktes von 8 bis 10 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

## Beispiel c

20 Gewichtsteile der Verbindung des Beispiels 8 werden in einer Mischung gelöst, die aus 60 Gewichtsteilen Cyclohexanon, 30 Gewichtsteilen Isobutanol, 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßirge Dispersion.

Beispiel d

20 Gewichtsteile der Verbindung des Beispiels 10 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

Beispiel e

20 Teile der Verbindung des Beispiels 29 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Beispiel f

Zur Herstellung eines öligen Holzschutzmittels mit 1% Wirkstoff wird zunächst 1 Teil (Gewichtsteil) des Wirkstoffs 31 unter leichtem Erwärmen in 55 Teilen einer aromatenreichen Benzinfraktion gelöst. Anschließend werden 10 Teile eines Alkydharzes zugefügt und bei Raumtemperatur mit Testbenzin auf 100 Teile ergänzt.

In entsprechender Weise werden ölige Holzschutzmittel mit 0,25 bis 5 Gew.% des Wirkstoffs hergestellt.

Zur Herstellung wasserabweisender Imprägnieranstriche können den öligen Holzschutzmitteln sog. "water repellents" zugesetzt werden. Geeignete Substanzen sind beispielsweise Zinkstearat, Aluminiumstearat, Wachse. Ferner können zur Erzielung von Farbeffekten anorganische oder organische Pigmente in die Formulierungen eingearbeitet werden.

Zum Schutz des Holzes gegen Pilzbefall werden üblicherweise 50 bis 200 ml der angeführten öligen Holzschutzmittel je $m^2$ Holzoberfläche durch Streichen, Spritzen oder Tauchen aufgebracht.

**Patentansprüche für die Vertragsstaaten: BE CH DE FR GB IT LI NL SE**

1. Azolylmethyloxirane der allgemeinen Formel I

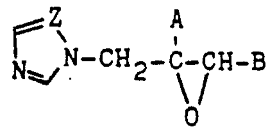

(I)

in welcher

A und B gleich oder verschieden sind und unabhängig voneinander Naphthyl, Biphenyl oder Phenyl bedeuten, wobei der Phenylrest durch Halogen, Nitro, Alkyl, Alkoxy oder Halogenalkyl mit jeweils 1 bis 4 Kohlenstoffatomen, Phenoxy sowie den Phenylsulfonylrest substituiert sein kann,

Z für die CH-Gruppe oder ein Stickstoffatom steht, sowie deren physiologisch bzw. für Pflanzen verträgliche Salze.

2. Verfahren zur Herstellung der Azolylmethyloxirane der Formel I gemäß Anspruch 1, dadurch gekennzeichnet, daß man

a) eine Verbindung der Formel II

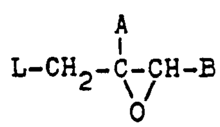

(II)

in welcher A und B die angegebenen Bedeutungen haben und L eine nukleophil substituierbare Abgangsgruppe darstellt, mit einer Verbindung der Formel III

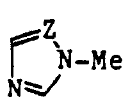

(III)

in der Me ein Wasserstoffatom oder ein Metallatom bedeutet und Z für eine CH-Gruppe oder ein Stickstoffatom steht, zur Umsetzung bringt, oder

b) eine Verbindung der Formel II, in der A und B die oben angegebenen Bedeutungen haben und L für eine Hydroxygruppe steht, mit einer Verbindung der Formel IV

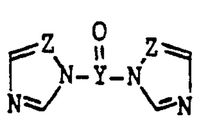

(IV),

in welcher Z die angegebene Bedeutung hat und Y ein Kohlenstoff- oder ein Schwefelatom bedeutet, zur Reaktion bringt oder

c) eine Verbindung der Formel V

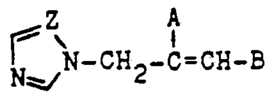

(V),

in welcher Z, A und B die angegebene Bedeutung haben, expoxidiert oder

d) eine Verbindung der Formel VI

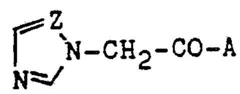

(VI),

in welcher Z und A die angegebene Bedeutung haben, mit einer Verbindung der allgemeinen Formel VII

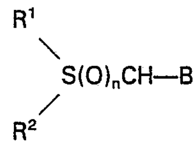

(VII),

in welcher B die angegebene Bedeutung hat, $R^1$ sowie $R^2$, die gleich oder verschieden voneinander sein können, eine Methyl- oder Phenyl-Gruppe bedeuten und n Null oder Eins bedeutet, zur Reaktion bringt und die so erhaltenen Verbindungen gegebenenfalls in ihre Salze mit physiologisch bzw. für Pflanzen verträglichen Säuren überführt.

3. Arzneimittel, enthaltend eine Verbindung gemäß Anspruch 1.

4. Verbindung der Formel I gemäß Anspruch 1 zur Verwendung bei der Bekämpfung von Krankheiten.

5. Fungizides Mittel, enthaltend eine Verbindung der Formel I gemäß Anspruch 1.

6. Fungizides Mittel, enthaltend eine Verbindung der Formel I gemäß Anspruch 1 und inerte Zusatzstoffe.

7. Verfahren zur Bekämpfung von Pilzen, dadurch gekennzeichnet, daß man eine fungizid wirksame Menge einer Verbindung der Formel I gemäß Anspruch 1 auf die Pilze oder auf durch Pilzbefall bedrohte Materialien, Pflanzen oder deren Anbauflächen oder Saatgüter einwirken läßt.

8. Verbindung der Formel

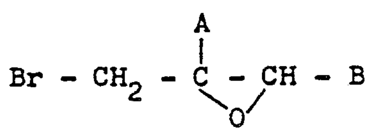

(II),

worin A und B die in Anspruch 1 gegebene Bedeutung haben.

**Patentansprüche für den Vertragsstaat: AT**

1. Verfahren zur Herstellung von Azolylmethyloxiranen der Formel I

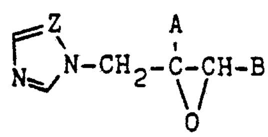

(I)

in welcher

A und B gleich oder verschieden sind und unabhängig voneinander Naphthyl, Biphenyl oder Phenyl bedeuten, wobei der Phenylrest durch Halogen, Nitro, Alkyl, Alkoxy oder Halogenalkyl mit jeweils 1 bis 4 Kohlenstoffatomen, Phenoxy sowie den Phenylsulfonylrest substituiert sein kann,

Z für die CH-Gruppe oder ein Stickstoffatom steht, sowie deren physiologisch verträgliche Salze, dadurch gekennzeichnet, daß man

a) eine Verbindung der Formel II

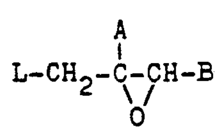

(II)

in welcher A und B die angegebenen Bedeutungen haben und L eine nukleophil substituierbare Abgangsgruppe darstellt, mit einer Verbindung der Formel III

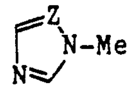 (III)

in der Me ein Wasserstoffatom oder ein Metallatom bedeutet und Z für eine CH-Gruppe oder ein Stickstoffatom steht, zur Umsetzung bringt, oder

b) eine Verbindung der Formel II, in der A und B die oben angegebenen Bedeutungen haben und L für eine Hydroxygruppe steht, mit einer Verbindung der Formel IV

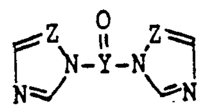 (IV),

in welcher Z die angegebene Bedeutung hat und Y ein Kohlenstoff- oder ein Schwefelatom bedeutet, zur Reaktion bringt oder

c) eine Verbindung der Formel V

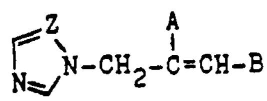 (V),

in welcher Z, A und B die angegebene Bedeutung haben, epoxidiert oder

d) eine Verbindung der Formel VI

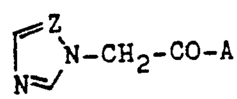 (VI),

in welcher Z und A die angegebene Bedeutung haben, mit einer Verbindung der allgemeinen Formel VII

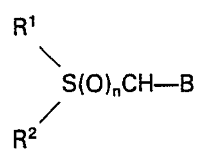 (VII),

in welcher B die angegebene Bedeutung hat, $R^1$ sowie $R^2$, die gleich oder verschieden voneinander sein können, eine Methyl- oder Phenyl-Gruppe bedeuten und n Null oder Eins bedeutet, zur Reaktion bringt und die so erhaltenen Verbindungen gegebenenfalls in ihre Salze mit physiologisch verträglichen Säuren überführt.

2. Fungizides Mittel, enthaltend ein Verbindung der Formel I, gemäß Anspruch 1 und inerte Zusatzstoffe.

3. Verfahren zur Bekämpfung von Pilzen, dadurch gekennzeichnet, daß man ein fungizid wirksame Menge einer Verbindung der Formel I, gemäß Anspruch 1 auf die Pilze oder auf durch Pilzbefall bedichte Materialen, Pflanzen oder deren Anbauflächen oder Saatgüter einwirken läßt.

4. Verfahren zur Herstellung eine Verbindung der Formel II

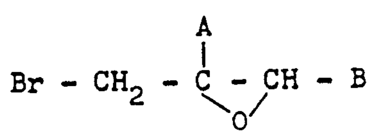 (II),

worin A und B die im Anspruch 1 gegebene Bedeutung haben dadurch gekennzeichnet, daß man ein Olefin der Formel IX

$$Br—CH_2—CA=CH—B \quad IX,$$

worin A und B die angegebene Bedeutung besitzen, epoxidiert.

**Revendications pour les Etats contractants: BE CH DE FR GB IT LI NL SE**

1. Azolyl-méthyl-oxirannes de la formule générale I

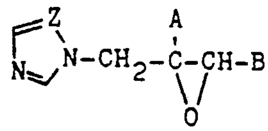

(I)

dans laquelle A et B, qui peuvent être identiques ou différents, désignent chacun, indépendamment l'un de l'autre, un groupe naphtyle, biphényle ou phényle, ce dernier pouvant porter des substituants halogène, nitro, alkyle, alcoxy ou halo-alkyle en $C_1$ à $C_4$, phénoxy ou phényl-sulfonyle et Z représente un groupe —CH ou un atome d'azote, ainsi que les sels physiologiquement et(ou) phyto-compatibles.

2. Procédé de préparation d'azolyl-méthyl-oxirannes de la formule I suivant la revendication 1, caractérisé en ce que:

a) on fait réagir un composé de la formule II

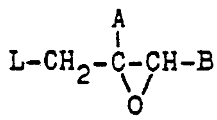

(II)

dans laquelle A et B possèdent les significations définies et L désigne un groupe éliminable pouvant porter un substituant nucléophile, et un composé de la formule III

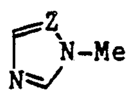

(III)

dans laquelle Me désigne un atome d'hydrogène ou un atome d'un métal, tandis que Z représente un groupe —CH ou un atome d'azote; ou

b) on fait réagir un composé de la formule II, dans laquelle A et B possèdent les significations définies plus haut et L désigne un groupe oxhydryle, avec un composé de la formule IV

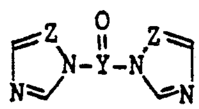

(IV),

dans laquelle Z possède la signification définie et Y désigne un atome de carbone ou un atome de soufre; ou

c) on soumet à une époxydation un composé de la formule V

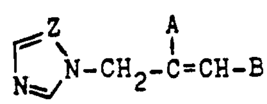

(V),

dans laquelle Z, A et B possèdent la signification définie; ou

d) on fait réagir un composé de la formule VI

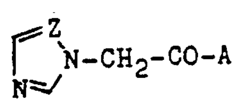

(VI),

dans laquelle Z et A possèdent la signification définies, avec un composé de la formule VII

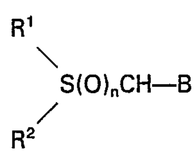

(VII),

dans laquelle B possède la signification définie et $R^1$ et $R^2$, qui peuvent être identiques ou différents,

désignent chacun un radical méthyle ou un groupe phényle et n vaut 0 ou 1, les produits réactionnels obtenus pouvant, si on le désire, être transformés en leurs sels d'acides physioligiquement et(ou) phyto-compatibles.

3. Médicament, contenant un composé suivant la revendication 1.

4. Composé de la formule I suivant la revendication 1, utilisé pour traiter des maladies.

5. Composition fongicide, contenant un composé de la formule I suivant la revendication 1.

6. Composition fongicide, contenant un composé de la formule I suivant la revendication 1, ainsi que des additifs inertes.

7. Procédé de lutte contre des champignons, caractérisé en ce que l'on fait agir sur les champignons ou sur les matériaux, plantes, semences ou surfaces, risquant d'être infestés par les champignons, une quantité efficace du point de vue fongicide d'un composé de la formule I suivant la revendication 1.

8. Composé de la formule

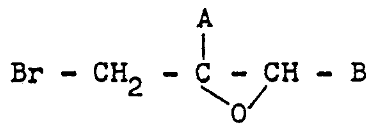

(II),

dans laquelle A et B possèdent la signification définie dans la revendication 1.

**Revendications pour l'Etat contractant: AT**

1. Procédé de préparation d'azolyl-méthyl-oxirannes de la formule I

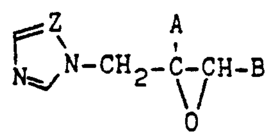

(I)

dans laquelle A et B, qui peuvent être identiques ou différents, désignent chacun, indépendamment l'un de l'autre, un groupe naphtyle, biphényle ou phényle, ce dernier pouvant porter des substituants halogène, nitro, alkyle, alcoxy ou halo-alkyle en $C_1$ à $C_4$, phénoxy ou phényl-sulfonyle et Z représente un groupe —CH ou un atome d'azote, ainsi que des sels physiologiquement compatibles de ceux-ci, caractérisé en ce que:

a) on fait réagir un composé de la formule I

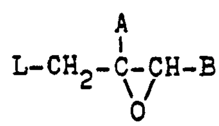

(II)

dans laquelle A et B possèdent les significations définies et L désigne un groupe éliminable pouvant porter un substituant nucléophile, et un composé de la formule III

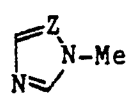

(III)

dans laquelle Me désigne un atome d'hydrogène ou un atome d'un métal, tandis que Z représente un groupe —CH ou un atome d'azote; ou

b) on fait réagir un composé de la formule II, dans laquelle A et B possèdent les significations définies plus haut et L désigne un groupe oxhydryle, avec un composé de la formule IV

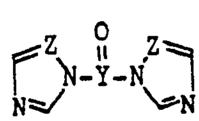

(IV),

dans laquelle Z possède la signification définie et Y désigne un atome de carbone ou un atome de soufre; ou

c) on soumet à une époxydation un composé de la formule V

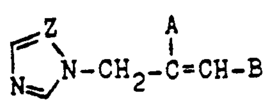

(V),

dans laquelle Z, A et B possèdent la signification définie; ou

d) on fait réagir un composé de la formule VI

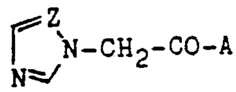 (VI),

dans laquelle Z et A possèdent la signification définies, avec un composé de la formule VII

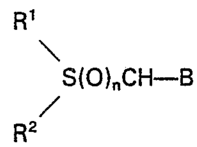 (VII),

dans laquelle B possède la signification définie et $R^1$ et $R^2$, qui peuvent être identiques ou différents, désignent chacun un radical méthyle ou un groupe phényle et n vaut 0 ou 1,
les produits réactionnels obtenus pouvant, si on le désire, être transformés en leurs sels d'acides physioligiquement compatibles.

2. Composition fongicide, contenant un composé de la formule I suivant la revendication 1 et des additifs inertes.

3. Procédé de lutte contre des champignons, caractérisé en ce que l'on fait agir sur les champignons ou sur les matériaux, plantes, semences ou surfaces, risquant d'être infestés par les champignons, une quantité efficace du point de vue fongicide d'un composé de la formule I suivant la revendication 1.

4. Procédé de préparation d'un composé de la formule II

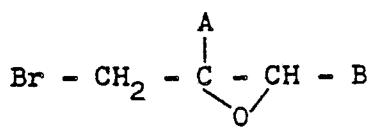 (II),

dans laquelle A et B possèdent la signification définie dans la revendication 1, caractérisé en ce que l'on soumet à une époxydation une oléfine de la formule IX

$$Br—CH_2—CA=CH—B \quad (IX),$$

dans laquelle A et B possèdent la signification définie.

**Claims for the Contracting States: BE CH DE FR GB IT LI NL SE**

1. An azolylmethyloxirane of the general formula I

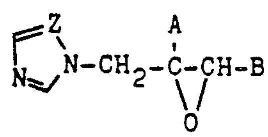 (I)

where
A and B are identical or different and, independently of one another, are naphthyl, biphenyl or phenyl, it being possible for the phenyl radical to be substituted by halogen or nitro, or by alkyl, alkoxy or haloalkyl, each of 1 to 4 carbon atoms, or by phenoxy or phenylsulfonyl, and
Z is CH or nitrogen,
or a physiologically tolerated or plant-tolerated salt thereof.

2. A process for the preparation of an azolylmethyloxirane of the formula I as claimed in claim 1, wherein
(a) a compound of the formula II

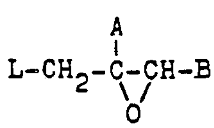 (II)

where A and B have the above meanings and L is a leaving group which can undergo nucleophilic

substitution, is reacted with a compound of the formula III

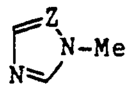 (III)

where Me is a hydrogen atom or a metal atom, and Z is CH or nitrogen, or

(b) a compound of the formula II, where A and B have the above meanings and L is a hydroxyl group, is reacted with a compound of the formula IV

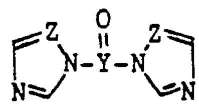 (IV),

where Z has the above meanings and Y is carbon or sulphur, or

(c) a compound of the formula V

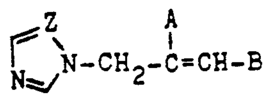 (V),

where Z, A and B have the above meanings, is epoxidized, or

(d) a compound of the formula VI

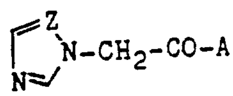 (VI),

where Z and A have the above meanings, is reacted with a compound of the general formula VII

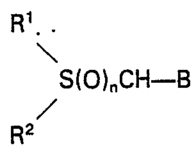 (VII),

where B has the above meanings, $R^1$ and $R^2$, which may be identical or different, are each methyl or phenyl, and n is 0 or 1, and, if desired, the compound thus obtained is converted into a salt with a physiologically tolerated or plant-tolerated acid.

3. A pharmaceutical containing a compound as claimed in claim 1.

4. A compound of the formula I as claimed in claim 1 for use in combating disease.

5. A fungicidal agent containing a compound of the formula I as claimed in claim 1.

6. A fungicidal agent containing a compound of the formula I as claimed in claim 1, and inert additives.

7. A process for combating fungi, wherein a fungicidally effective amount of a compound of the formula I as claimed in claim 1 is allowed to act on the fungi, or on materials, plants or areas on which they are planted, or seed threatened by fungal attack.

8. A compound of the formula

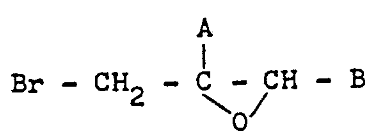 (II),

where A and B have the meanings given in claim 1.

**Claims for the Contracting State: AT**

1. A process for the preparation of a azolylmethyloxirane of the general formula I

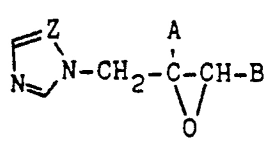 (I)

where

A and B are identical or different, and independently of one another, are naphthyl, biphenyl or phenyl, it being possible for the phenyl radical to be substituted by halogen or nitro, or by alkyl, alkoxy or haloalkyl, each of 1 to 4 carbon atoms, or by phenoxy or phenylsulfonyl, and Z is CH or nitrogen, or a physiologically tolerated salt thereof, wherein

(a) a compound of the formula II

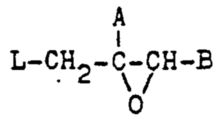 (II)

where A and B have the above meanings and L is a leaving group which can undergo nucleophilic substitution, is reacted with a compound of the formula III

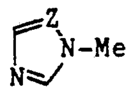 (III)

where Me is a hydrogen atom or a metal atom, and Z is CH or nitrogen, or

(b) a compound of the formula II, where A and B have the above meanings and L is a hydroxyl group, is reacted with a compound of the formula IV

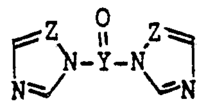 (IV),

where Z has the above meanings and Y is carbon or sulphur, or

(c) a compound of the formula V

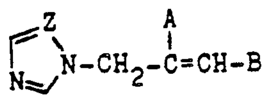 (V),

where Z, A and B have the above meanings, is epoxidized, or

(d) a compound of the formula VI

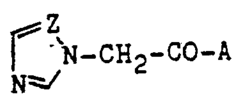 (VI),

where Z and A have the above meanings, is reacted with a compound of the general formula VII

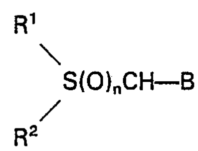 (VII),

where B has the above meanings, $R^1$ and $R^2$, which may be identical or different, are each methyl or phenyl, and n is 0 or 1, and, if desired, the compound thus obtained is converted into a salt with a physiologically tolerated acid.

2. A fungicidal agent containing a compound of the formula I as claimed in claim 1, and inert additives.

3. A process for combating fungi, wherein a fungicidally effective amount of a compound of the formula I as defined in claim 1 is allowed to act on the fungi, or on materials, plants or areas on which they are planted, or seed threatened by fungal attack.

4. A process for the preparation of a compound of the formula II

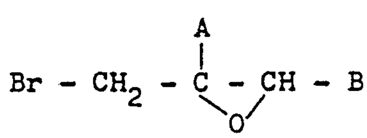 (II),

where A and B have the meanings given in claim 1, wherein an olefin of the formula IX

$$Br—CH_2—CA{=}CH—B \qquad (IX),$$

where A and B have the above meanings, is epoxidized.